Europäisches Patentamt

European Patent Office

Office européen des brevets

(11) Veröffentlichungsnummer: **0 282 870 B1**

(12) **EUROPÄISCHE PATENTSCHRIFT**

(45) Veröffentlichungstag der Patentschrift: **11.03.92**

(21) Anmeldenummer: **88103537.2**

(22) Anmeldetag: **07.03.88**

(51) Int. Cl.⁵: **A61B 6/03**, H05G 1/26

Die Akte enthält technische Angaben, die nach dem Eingang der Anmeldung eingereicht wurden und die nicht in dieser Patentschrift enthalten sind.

(54) **Computertomograph.**

(30) Priorität: **20.03.87 DE 8704182 U**

(43) Veröffentlichungstag der Anmeldung:
**21.09.88 Patentblatt 88/38**

(45) Bekanntmachung des Hinweises auf die Patenterteilung:
**11.03.92 Patentblatt 92/11**

(84) Benannte Vertragsstaaten:
**DE FR**

(56) Entgegenhaltungen:
**US-A- 4 220 860**
**US-A- 4 559 639**

**PATENT ABSTRACTS OF JAPAN, Band 5, Nr. 34 (E-48)[706], 4. März 1981, Seite 29 E 48; & JP-A-55 159 596 (SHIMAZU SEISAKUSHO K.K.) 11-12-1980**

**PATENT ABSTRACTS OF JAPAN, Band 6, Nr. 87 (E-108)[965], 25. Mai 1982, Seite 37 E 108; & JP-A-57 21 100 (TOKYO SHIBAURA DENKI K.K.) 03-02-1982**

(73) Patentinhaber: **SIEMENS AKTIENGESELL-SCHAFT**
**Wittelsbacherplatz 2**
**W-8000 München 2(DE)**

(72) Erfinder: **Göthert, Dieter**
**Nr. 115**
**W-8551 Leutenbach(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft einen Computertomographen mit einer Meßeinheit aus einer Röntgenstrahlenquelle und einem Strahlenempfänger, bei dem die Röntgenstrahlenquelle ein fächerförmiges Nutzstrahlenbündel aussendet, das auf dem aus einer Reihe von Einzeldetektoren bestehenden Strahlenempfänger auftrifft, von denen jeder ein der empfangenen Strahlenintensität entsprechendes elektrisches Signal bildet, bei dem Mittel zur Drehung des Nutzstrahlenbündels um das Meßfeld zur Durchstrahlung des Meßfeldes unter verschiedenen Richtungen vorgesehen sind, bei dem eine Meßwertverarbeitungseinheit vorhanden ist, der die Ausgangssignale der Einzeldetektoren, die bei den verschiedenen Durchstrahlungsrichtungen erzielt werden, zugeführt werden und die daraus die Schwächungswerte vorbestimmter Punkte in der durchstrahlten Schicht des Aufnahmeobjektes bestimmt und bei dem eine Bildwiedergabevorrichtung zur bildlichen Wiedergabe der berechneten Schwächungswerte vorhanden ist.

Bei einem Computertomographen dieser Art wird ein Bild der druchstrahlten Schicht des Untersuchungsobjektes aus den bei verschiedenen Durchstrahlungsrichtungen gewonnenen Ausgangssignalen der Einzeldetektoren rekonstruiert. Voraussetzung für ein artefaktfreies Bild ist dabei, daß der Fokus im Bezug auf den Strahlenempfänger eine vorbestimmte Sollage einnimmt. In der Praxis kann es nun vorkommen, daß sich der Fokus insbesondere in Längsrichtung der Patientenliege (z-Richtung) geringfügig gegenüber der Sollage verstellt, und zwar aufgrund der Erwärmung der Röntgenröhre. Zur Kompensation der dadurch verursachten Signalbeeinflussung ist in der US-PS 4,559,639 vorgeschlagen, eine Signalkorrektur aufgrund von Veränderungen der Fokuslage, die mit Hilfe von Meßdetektoren erfaßt wird, vorzunehmen.

Der Erfindung liegt die Aufgabe zugrunde, einen Computertomographen der eingangs genannten Art so auszubilden, daß eine Korrektur der Veränderung der Fokuslage erfolgt, ohne daß hierzu Meßdetektoren für die tatsächliche Erfassung der jeweiligen Fokuslage erforderlich sind.

Diese Aufgabe ist erfindungsgemäß dadurch gelöst, daß ein Rechner vorhanden ist, der aus den Betriebswerten die Fokusverlagerung des Nutzstrahlenbündels bestimmt und ein entsprechendes Signal der Meßwertverarbeitungseinheit zuführt. Bei dem erfindungsgemäßen Computertomographen ist davon ausgegangen, daß aus den Betriebswerten des Computertomographen auf die jeweils vorhandene Temperatur der Drehanode der Röntgenstrahlenquelle geschlossen werden kann und daß zwischen dieser Temperatur und der jeweiligen Fokuslage, insbesondere in z-Richtung, ein funktioneller Zusammenhang besteht. Demgemäß kann aus den Betriebswerten des Computertomographen, zu denen die Aufnahmewerte und die jeweils verstrichene Aufnahmezeit gehören, auf die jeweilige Fokuslage geschlossen werden. Ein entsprechendes Signal bewirkt eine Korrektur zur Erzielung artefaktfreier Bilder. Der funktionelle Zusammenhang zwischen der gerechneten Temperatur und der Position des Fokus in z-Richtung wird einmal für eine typische Anordnung gemessen und abgespeichert.

Die Erfindung ist nachfolgend anhand eines in der Zeichnung dargestellten Ausführungsbeispiels näher erläutert. Es zeigen:

Fig. 1     einen Computertomographen nach der Erfindung, und

Fig. 2     eine Ansicht der für die Erfindung wesentlichen Teile des Computertomographen gemäß Fig. 1 in Richtung des Pfeiles II.

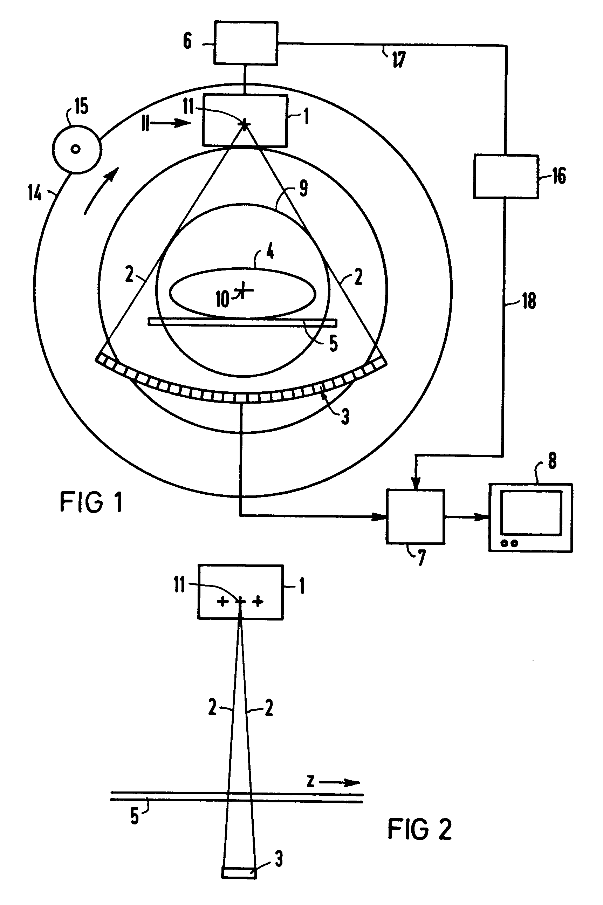

Der Computertomograph gemäß Fig. 1 weist eine Meßeinheit aus einer Röntgenstrahlenquelle 1, die ein fächerförmiges Röntgenstrahlenbündel 2 aussendet, und einem Strahlenempfänger 3 auf, welcher aus einer Reihe von Einzeldetektoren, z.B. aus 512 Einzeldetektoren besteht. Der zu untersuchende Patient 4 liegt auf einer Patientenliege 5. Zur Abtastung des Patienten 4 wird die Meßeinheit 1, 3 um ein Meßfeld 9, in dem der Patient 4 liegt, um 360° gedreht. Die Drehachse ist mit 10 bezeichnet. Dabei wird die Röntgenstrahlenquelle 1, die von einem Röntgengenerator 6 gespeist wird, gepulst oder mit Dauerstrahlung betrieben. Bei vorbestimmten Winkelpositionen der Meßeinheit 1, 3 werden Sätze von Daten erzeugt, die vom Strahlenempfänger 3 einem Rechner 7 zugeführt werden, welcher aus den erzeugten Datensätzen die Schwächungskoeffizienten vorbestimmter Bildpunkte berechnet und auf einem Sichtgerät 8 bildlich wiedergibt. Auf dem Sichtgerät 8 erscheint demgemäß ein Bild der durchstrahlten Schicht des Patienten 4.

Die Änderung der Richtung des Nutzstrahlenbündels 2 erfolgt durch Drehung eines Drehkranzes 14, auf dem die Röntgenstrahlenquelle 1 und der Strahlenempfänger 3 angeordnet sind, mit Hilfe einer Drehvorrichtung 15.

Die Fig. 2 zeigt, daß sich der Fokus 11 aufgrund der Erwärmung der Röntgenstrahlenquelle 1 geringfügig in z-Richtung verlagern kann. Dabei ist für einen bestimmten Computertomographen ein fester funktioneller Zusammenhang zwischen der jeweiligen Fokuslage und der Temperatur der Röntgenstrahlenquelle 1, nämlich der Drehanode dieser Röntgenstrahlenquelle 1, gegeben. Dieser funktionelle Zusammenhang ist in einem Rechner 16 abgespeichert, der am Eingang 17 Signale erhält, die den Betriebswerten des Computertomographen entsprechen. Der Rechner 16 bestimmt aus den Signalen am Eingang 17 die jeweilige Temperatur der Röntgenstrahlenquelle 1 und daraus die jeweilige Fokuslage. Ein der jeweiligen Fokuslage entsprechendes Signal wird auf einer Leitung 18 dem Rechner 7 zugeführt und bewirkt dort eine Korrektur in der Weise, daß das auf dem Sichtgerät 8 wiedergegebene Bild der durchstrahlten Schicht des Patienten 4 frei von Artefakten ist, die durch Änderungen der Fokuslage aufgrund der Erwärmung der Röntgenstrahlenquelle 1 verursacht sind.

## Patentansprüche

1. Computertomograph mit einer Meßeinheit (1, 3) aus einer Röntgenstrahlenquelle (1) und einem Strahlenempfänger (3), bei dem die Röntgenstrahlenquelle (1) ein fächerförmiges Nutzstrahlenbündel (2) aussendet, das auf dem aus einer Reihe von Einzeldetektoren bestehenden Strahlenempfänger (3) auftrifft, von denen jeder ein der empfangenen Strahlenintensität entsprechendes elektrisches Signal bildet, bei dem Mittel (14, 15) zur Drehung des Nutzstrahlenbündels (2) um das Meßfeld (9) zur Durchstrahlung des Meßfeldes (9) unter verschiedenen Richtungen vorhanden sind, bei dem eine Meßwertverarbeitungseinheit (7) vorhanden ist, der die Ausgangssignale der Einzeldetektoren, die bei den verschiedenen Durchstrahlungsrichtungen erzielt werden, zugeführt werden und die daraus die Schwächungswerte vorbestimmter Punkte in der durchstrahlten Schicht des Aufnahmeobjektes (4) bestimmt und bei dem eine Bildwiedergabevorrichtung (8) zur bildlichen Wiedergabe der berechneten Schwächungswerte vorhanden ist, **dadurch gekennzeichnet,** daß ein Rechner (16) vorhanden ist, der über seinen Eingang (17) zur Zuführung von den Betriebswerten des Computertomographen entsprechenden Signalen am Röntgengenerator (6) angeschlossen ist, und der über eine Leitung (18) an der Meßwertverarbeitungseinheit (7) angeschlossen ist und dieser ein aus den Betriebswerten berechnetes, der Fokusverlagerung des Nutzstrahlenbündels (2) entsprechendes Korrektursignal zuführt.

## Claims

1. Computer tomography apparatus having a measuring unit (1, 3) comprising an X-ray source (1) and a ray receiver (3), wherein the X-ray source (1) emits a fan-shaped working beam (2), which strikes the ray receiver (3) consisting of a series of individual detectors, each of which forms an electrical signal corresponding to the received ray intensity, wherein means (14, 15) are present for the rotation of the working beam (2) round the field of measurement (9) for the irradiation of the field of measurement (9) in various directions, wherein a measured-value processing unit (7) is present, to which there are supplied the output signals of the individual detectors attained with the various irradiation directions, and which determines therefrom the attenuation values of predetermined points in the irradiated layer of the object to be recorded (4) and wherein an image reproduction device (8) is present for the graphic reproduction of the calculated attenuation values, characterized in that a computer (16) is present, which is attached to the X-ray generator (6) by means of its input (17) for the supply of signals corresponding to the operational values of the computer tomography apparatus, and which is attached to the measured-value processing unit (7) by means of a line (18) and supplies to it a correction signal calculated from the operational values and corresponding to the displacement of the focus of the working beam (2).

## Revendications

1. Tomodensitomètre comportant une unité de mesure (1,3) formée par une source (1) de rayons X et un récepteur (3) de rayons X, et dans lequel la source (1) de rayons X émet un faisceau de rayonnement utile (2) en forme d'éventail, qui rencontre le récepteur de rayonnement (3) constitué par une rangée de détecteurs individuels, dont chacun forme un signal électrique qui correspond à l'intensité de rayonnement reçue, et dans lequel il est prévu des moyens (14,15) pour faire tourner le faisceau de rayons utiles (2) autour du champ de mesure (9) pour irradier ce dernier dans différentes directions, et dans lequel il est prévu une unité (7) de traitement des valeurs de mesure, à laquelle sont envoyés les signaux de sortie des différents détecteurs, qui sont obtenus dans les différentes directions d'irradiation et qui détermine, à partir de là, les valeurs d'affaiblissement de points prédéterminés dans la couche irradiée de l'objet de prise de vues (4), et dans lequel il est prévu un dispositif (8) de reproduction d'images, servant à reproduire sous la forme d'une image les valeurs d'affaiblissement calculées, caractérisé par le fait qu'il est prévu un ordinateur (16) qui est raccordé, par l'intermédiaire de son entrée (17), pour l'envoi de signaux correspondant aux valeurs de service du tomodensitomètre, au générateur (6) de rayons X, et qui est raccordé, par l'intermédiaire d'une ligne (18) à l'unité (7) de traitement des valeurs de mesure et envoie à cette dernière un signal de correction, calculé à partir des valeurs de service et correspondant au décalage du foyer du faisceau de rayons utiles (2).

EP 0 282 870 B1

FIG 1

FIG 2

5